# EUROPEAN PATENT SPECIFICATION

(11) **EP 2 574 274 B1**
(45) Date of publication and mention of the grant of the patent: **08.04.2020**
(21) Application number: 12186037.3
(22) Date of filing: 26.09.2012
(51) Int. Cl.: A61B 5/00, A61B 8/12

(54) **Motor drive apparatus and imaging apparatus for diagnosis**
Motorantriebsvorrichtung und Abbildungsvorrichtung zur Diagnose
Appareil de commande de moteur et appareil d'imagerie pour diagnostic

(30) Priority: 28.09.2011 JP 2011211757
(43) Date of publication of application: 03.04.2013
(73) Proprietor: Terumo Kabushiki Kaisha, Tokyo (JP)
(72) Inventor: Horiike, Toyokazu, Fujinomiya-city, Shizuoka (JP); Fukuda, Yozo, Fujinomiya-city, Shizuoka (JP)
(74) Representative: Dossmann, Gérard

(56) References cited:
- JP-A- 2000 116 654
- JP-A- 2005 237 980
- US-A1- 2005 203 699
- US-A1- 2007 232 892

## Description

The present invention generally relates to a motor drive apparatus and an imaging apparatus for diagnosis.

State of the art imaging apparatuses are for example known from JP2005237980A and JP2000116654A.

In the past, for purposes of diagnosing arteriosclerosis, for performing diagnosis before a surgical operation at the time of intravascular diagnosis by a high-functional catheter such as a balloon catheter or a stent and the like, or to confirm the result after a surgical operation, there has been utilized an ultrasound imaging apparatus for diagnosis, an optical coherent tomography (OCT) apparatus as disclosed for example in U.S. Patent Application Publication No. 2012/0002928) or an optical frequency domain imaging (OFDI) apparatus utilizing wavelength sweep, which is an improved type of OCT apparatus. In this specification, the optical coherent tomography (OCT) apparatus and the optical frequency domain imaging (OFDI) apparatus utilizing wavelength sweep will be together referred to as "optical imaging apparatus for diagnosis" and further referred to as "imaging apparatus for diagnosis" also with inclusion of the ultrasound imaging apparatus for diagnosis.

Known ultrasound imaging apparatus for diagnosis include, for example, an intravascular ultrasound (IVUS) diagnostic apparatus. The intravascular ultrasound (IVUS) diagnostic apparatus radially operates an ultrasound probe unit, which is built-in with a transmitting and receiving unit composed of an ultrasound transducer, inside a blood vessel; receives a reflected wave (ultrasonic wave echo), which is reflected by biological tissue of a test subject, by the transmitting and receiving unit; and thereafter, visualizes a cross-sectional image of the blood vessel based on the intensity of the ultrasonic wave echo signal generated by applying a process of amplification, detection and the like.

In the optical imaging apparatus for diagnosis, an optical probe unit, having an imaging core mounted with an optical lens and an optical mirror (transmitting and receiving unit) at a distal end of an optical fiber, is inserted into the inside a tubular lumen of a biological tissue such as a blood vessel or the like, measurement light is emanated or emitted from the transmitting and receiving unit at the distal end to the biological tissue while rotating the imaging core, and concurrently, a radial scan inside a body lumen is carried out by light-receiving the reflected light from the biological tissue. Then, based on the interference light generated by making the light-received reflected light and the reference light interfere with each other, a cross-sectional image of the biological tissue is visualized or produced.

Generally, for the radial scan of the imaging core in the imaging apparatus for diagnosis, there is utilized a motor drive apparatus referred to as a scanner & pull-back unit. The scanner & pull-back unit is constituted by a scanner unit and a pull-back unit, and the probe unit is mounted detachably at the distal end portion of the scanner unit.

Here, on an occasion of the radial scan of the imaging core, a procedure is used in which the scanner unit is once moved rearward, mounting of the probe unit is carried out under a situation that the mounting space on the distal end side of the scanner unit is secured and thereafter, a pull-back operation is carried out under a situation that the scanner unit is moved up to the forward end.

Also, in case of carrying out visualization of the cross-sectional image once again after the visualization of the cross-sectional image of the biological tissue is completed by the pull-back operation, it is necessary to move the scanner unit lying at the backward end up to the forward end again.

In this manner, for the utilization of the scanner & pull-back unit, the operation of moving the scanner unit up to the forward end is indispensable. In the past, there was provided a forward button constituted such that the scanner unit moves a distance proportional to the pressing period of the button, wherein the scanner unit is moved up to the forward end by a configuration in which the user operates that button.

In the case of the forward button, it is difficult to make an accurate stop at the forward end and, for example, in a case in which the timing of releasing the press of the forward button is delayed, it sometimes happens that the abutment unit of the probe unit collides with the sheath proximal-end, thereby causing damage to the probe unit.

On the other hand, by pressing the forward button under a situation in which the moving speed of the probe unit is slowed down, it becomes possible to avoid the collision, but in this case, it takes time for the movement up to the forward end to occur, thus reducing the working efficiency of the user.

Also, if the timing of releasing the press of the forward button is too early for the purpose of avoiding the collision, the scanner unit stops at a position spaced a distance from the forward end. A situation thus occurs in which it becomes impossible to secure a sufficient amount of pull-back.

The motor drive apparatus disclosed here is mounted with a probe unit including a transmitting and receiving unit which carries out signal transmission and reception continuously. The motor drive apparatus includes: a scanner unit for rotating the transmitting and receiving unit, a scanner moving unit for axially operating or moving the scanner unit to move the transmitting and receiving unit in the axial direction inside a body lumen, and a detection unit for detecting that the scanner unit has reached a position apart by a predetermined distance from the forward end position of the scanner unit in case of operating the scanner unit axially toward the forward direction, wherein the scanner moving unit includes a control unit which carries out speed control based on a first speed set value until the detection by the detection unit in case of operating the scanner unit axially toward the forward direction, and which carries out the speed control based on a second speed set value smaller than the first speed set value after the detection by the detection unit.

The motor drive apparatus is able to carry out the movement of the scanner unit toward the forward end rather accurately and also efficiently without damaging the probe unit in the scanner & pull-back unit of the imaging apparatus for diagnosis. In a scanner & pull-back unit of an imaging apparatus for diagnosis, it is possible to carry out the axial movement of the scanner unit toward the forward end (in the forward direction) rather accurately and also efficiently without damaging the probe unit.

According to another aspect, a motor drive apparatus is connected to a probe unit that includes a transmitting and receiving unit which continuously transmits signals reflected as reflected signals and which receives the reflected signals, wherein the motor drive apparatus comprises: a scanner unit connected to and axially movable with the transmitting and receiving unit, and configured to rotate the transmitting and receiving unit; a motor operatively connected to the scanner unit and configured to axially move the scanner unit in a forward direction toward a forward-most end position of the scanner unit at which the axial movement of the scanner unit in the forward direction is stopped, the axial movement of the scanner unit also axially moving the transmitting and receiving unit; a sensor configured to detect that the scanner unit axially moving in the forward direction has reached a predetermined position at which the scanner unit is spaced away from the forward-most end position by a predetermined distance and which outputs a signal indicating the scanner unit axially moving in the forward direction has reached the predetermined position; and a control unit operatively connected to the sensor and to the motor to control a moving speed at which the scanner unit axially moves in the forward direction. The control unit controls the moving speed of the scanner unit to axially move the scanner unit moving in the forward direction at a first speed before the scanner unit axially moving in the forward direction reaches the predetermined position, and reduces the moving speed of the scanner unit axially moving in the forward direction so that the scanner unit continues axially moving in the forward direction but at a speed less than the first speed when the control unit receives the signal from the sensor indicating that the scanner unit axially moving in the forward direction has reached the predetermined position.

Another aspect involves a method of controlling a scanner axially moving in a forward direction towards a forward-most position, wherein the scanner unit is connected to a probe unit that includes a transmitting and receiving unit which continuously carries out signal transmission and reception. The method comprises: axially moving the scanner unit in the forward direction toward the forward-most position at a first speed, with the transmitting and receiving unit moving together with the scanner unit; detecting that the scanner unit axially moving in the forward direction has reached a detection position spaced from the forward-most end position of the scanner unit by a predetermined distance when the scanner unit is operating in the axial forward direction; and reducing the speed at which the scanner unit is axially moving in the forward direction when it is detected that the scanner unit axially moving in the forward direction has reached the detection position so that the scanner unit continues moving towards the forward-most end position but at a second speed less than the first speed.
FIG. 1 is a perspective view of the outward-appearance constitution of an imaging apparatus for diagnosis disclosed here.
FIGS. 2A and 2B are views showing a construction of an optical probe unit forming a part of the imaging apparatus for diagnosis.
FIG. 3 is a perspective view of a scanner & pull-back unit forming a part of the imaging apparatus for diagnosis.
FIG. 4 is a somewhat schematic illustration of functional attributes and features for operating the scanner unit in the axial direction.
FIG. 5 is a flowchart showing operational aspects of an automatic forward-movement control process in the pull-back unit.
FIGS. 6A to 6C are views showing an aspect of the axial movement operation of the scanner unit in the automatic forward-movement control process.

Set forth below is a detailed description of one embodiment of the imaging apparatus for diagnosis representing an example of the imaging apparatus for diagnosis and associated motor drive apparatus disclosed here.

### Outward-Appearance Constitution of Optical Imaging Apparatus for Diagnosis

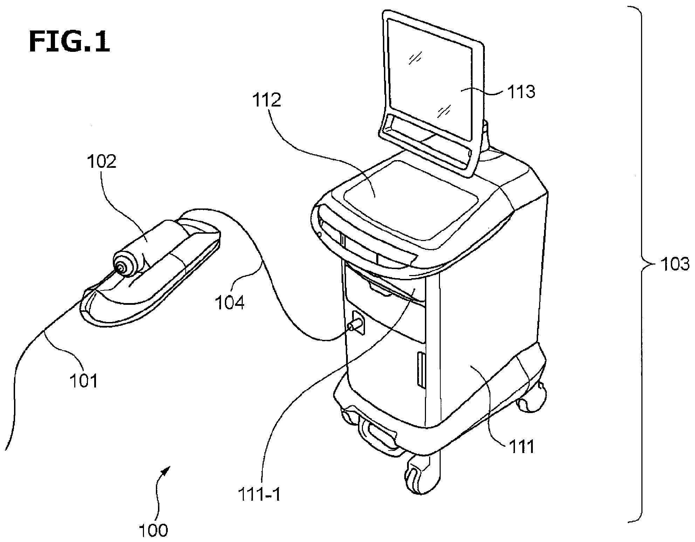

First, there will be explained an optical imaging apparatus for diagnosis provided with a scanner & pull-back unit. FIG. 1 illustrates the outward-appearance constitution (construction or configuration) of an optical imaging apparatus for diagnosis (optical coherent tomography (OCT) apparatus or optical frequency domain imaging (OFDI) apparatus utilizing wavelength sweep) 100 provided with a scanner & pull-back unit, which relates to this embodiment representing an example of the disclosure here.

As shown in FIG. 1, the optical imaging apparatus for diagnosis 100 is provided with an optical probe unit 101 as an example of a probe unit, a scanner & pull-back unit 102 and an operation control apparatus 103. The scanner & pull-back unit 102 and the operation control apparatus 103 are connected by a signal line 104.

The optical probe unit 101 is directly inserted into the inside of a tubular lumen of a biological tissue (blood vessel or the like), transmits transmitted measurement light continuously toward the biological tissue, and concurrently, is inserted with (has) an imaging core provided with a transmitting and receiving unit, which receives reflected light from the biological tissue continuously, at its distal end, in which the state of the biological tissue is measured using the imaging core.

The scanner & pull-back unit (motor drive apparatus) 102 is comprised of a scanner unit and a pull-back unit, i.e., scanner moving unit. The scanner unit is detachably mounted with the imaging core of the optical probe unit 101 and by driving a built-in motor, radial operation of the imaging core inserted into (positioned inside) the optical probe unit 101 occurs. Furthermore, the pull-back unit operates the scanner unit to perform the axial direction movement between a forward end position and a backward end position. For this movement of the scanner unit, the imaging core is moved in the axial direction of the optical probe unit 101. Also, the transmitting and receiving unit receives the reflected light and concurrently transmits the obtained reflected light to the operation control apparatus 103 through the signal line 104. A detailed description of the scanner & pull-back unit 102 is set forth later.

For carrying out the measurement, the operation control apparatus 103 is configured to permit inputting of various kinds of set values, to process data obtained by the measurement, and to display them as a cross-sectional image of the biological tissue.

In the operation control apparatus 103, a reference numeral 111 denotes a main body control unit in which interference light data is generated by making the reflected light which is obtained by the measurement (the measurement light transmitted at the biological tissue) and the reference light which is obtained by separating the measurement light interfere with each other and by concurrently processing line data generated based on the interference light data to generate the cross-sectional images, including a plurality of cross-sectional images extending toward the axial direction inside the body cavity. Reference numeral 111-1 denotes a printer & DVD recorder in which the processed result in the main body control unit 111 is printed, is stored as data and so on.

A reference numeral 112 denotes an operation panel at which the user carries out inputs of various kinds of set values and instructions. A reference numeral 113 denotes an LCD monitor as a display apparatus in which there are displayed a plurality of cross-sectional images of the biological tissue, which are generated in the main body control unit 111. Constitution of Optical Probe Unit

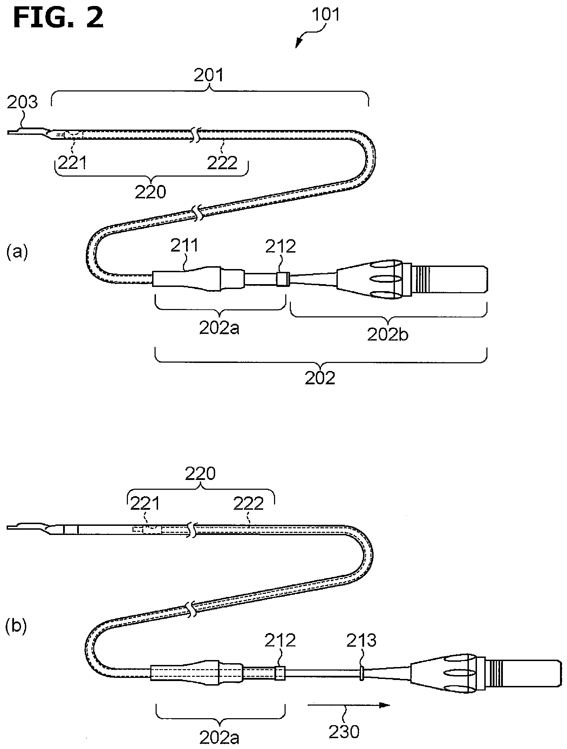

Set forth next with reference to FIG. 2 is a description of the overall construction of the optical probe unit 101. As shown in FIG. 2A, the optical probe unit 101 is constituted by an elongated catheter sheath 201 configured to be inserted inside a body lumen, and a connector unit 202 which is not inserted inside the body lumen in order to be operated by a user and which is arranged on the side of the user's hand. At the distal end of the catheter sheath 201, there is provided a tube 203 which constitutes a guide wire lumen, and the catheter sheath 201 is formed with a tubular lumen which continues from a connection portion with respect to the tube 203 to a connection portion with respect to the connector unit 202.

In the inside of the tubular lumen of the catheter sheath 201, there is inserted-through an imaging core 220 which is provided with a transmitting and receiving unit 221 for transmitting and receiving the measurement light and which is provided with an optical fiber cable in the inside thereof and is provided with a coil-shaped drive shaft 222 for transmitting a drive force for rotating that cable, approximately over the whole length of the catheter sheath 201.

The connector unit 202 is provided with a sheath connector 202a constituted integrally with the proximal end of the catheter sheath 201. The connector unit 202 also includes a drive shaft connector 202b in which is positioned a rotatable connector fixed to the drive shaft 222. The drive shaft connector 202b thus rotatably retains the rotatable connector and the proximal end of the drive shaft 222 on the inside.

At the boundary portion between the sheath connector 202a and the catheter sheath 201, there is provided an anti-kink protector 211. Depending on this configuration, a predetermined rigidity is maintained and it is possible to prevent bending (kinking) caused by a rapid change in the material property.

A sheath proximal end 212 at a boundary portion between the sheath connector 202a and the drive shaft connector 202b is fixed detachably on the pull-back unit side, which will described later when mounting the optical probe unit 101 on the scanner & pull-back unit 102. On the other hand, the proximal end of the drive shaft connector 202b is mounted detachably to a distal-end apparatus of a scanner unit which will be described later.

Thus, in a case in which the scanner unit carries out the pull-back operation, the drive shaft connector 202b moves in a straight line (axial) manner in the direction of the arrow 230 (backward direction) and the imaging core 220 is operated in the axial direction (see FIG. 2B).

An abutment unit 213 is provided on the distal end side of the drive shaft connector 202b and in a case in which the scanner unit carries out a forward-movement operation (in case of carrying out an axial movement operation in the direction opposite to the arrow 230), a situation arises in which the forward end position of the drive shaft connector 202b is defined by a mechanism in which the abutment unit 213 abuts the sheath proximal end 212.

### Whole Constitution of Scanner & Pull-back Unit

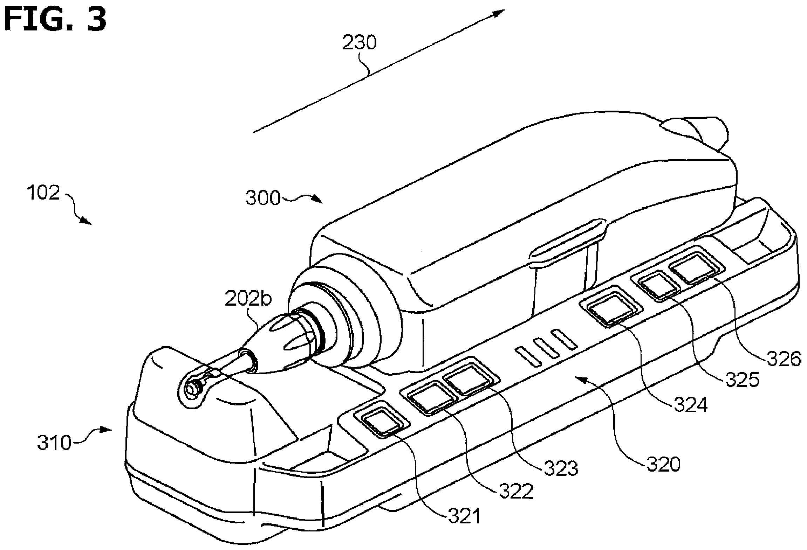

Set forth next with reference to FIG. 3 is a description of the construction of the scanner & pull-back unit 102 according to this embodiment disclosed by way of example. As shown in FIG. 3, the scanner & pull-back unit 102 is detachably mounted with the optical probe unit 101 (i.e., the optical probe unit 101 is detachably mountable on the scanner & pull-back unit 102) and is provided with a scanner unit 300 for rotating the imaging core 220 which is inserted into (positioned inside) the optical probe unit 101 and a pull-back unit (scanner moving unit) 310 which operates the imaging core 220 inserted into the optical probe unit 101 in the axial direction of a body lumen by operating or moving the scanner unit 300 in the direction of the arrow 230.

The scanner unit 300 includes a built-in motor for rotational operation for rotating the imaging core 220, which realizes a rotation speed of 9600rpm at the maximum. The pull-back unit 310 also includes a built-in motor for operating or moving the imaging core 220 in the axial direction inside the body lumen by operating the scanner unit 300 in the forward direction. The side surface (right side in the drawing) of the pull-back unit 310 is provided with an operation unit 320.

The operation unit 320 includes buttons for instructing the rotational operation and the axial operation of the scanner & pull-back unit 102, and various lamps for displaying the state inside the scanner & pull-back unit 102.

The operation unit 320 includes an automatic forward button 321 (instruction unit) which, when pressed or operated, moves the scanner unit 300 to the forward end automatically, a fine-forwarding button 322 which, during the period when this button is pressed or operated, moves the scanner unit 300 toward the forward direction at a lower speed or relatively low speed, and a fine-retreat button 323 which, during the period when this button is pressed or operated, moves the scanner unit 300 toward the retreating direction at a lower speed or relatively low speed.

The operation unit 320 further includes a low speed scan-start button 324, and by pressing this button, the imaging core 220 of the optical probe unit 101 starts rotating at a lower speed or relatively low speed (for example, 1800rpm). A high speed scan-start button 325 is also provided, and by pressing this button, the imaging core 220 of the optical probe unit 101 starts the rotation at a relatively high speed or higher speed (for example, 9600rpm). A reference numeral 326 denotes a pull-back button and the pressing of this button becomes possible in a case in which the imaging core 220 of the optical probe unit 101 rotates at a relatively high speed or higher speed, whereby pressing this button 326 causes the scanner unit 300 to be pulled-back at a predetermined speed.

### Functional Constitution for Axially Operating Scanner Unit

Set forth next is an explanation of operational or functional aspects for operating the scanner unit 300 axially in the scanner & pull-back unit 102.

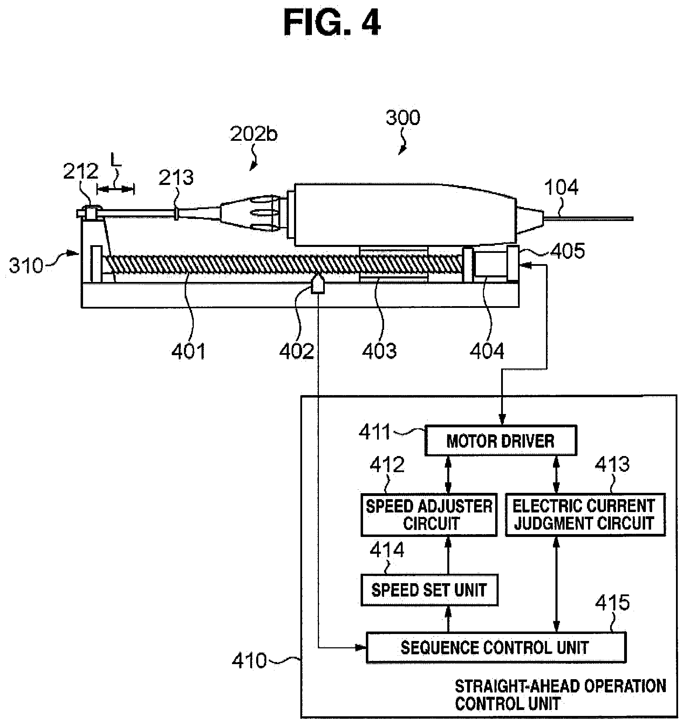

FIG. 4 schematically illustrates features associated with axially operating or moving the scanner unit 300. As shown in FIG. 4, movement (axial direction operation) of the imaging core 220 of the optical probe unit 101 in the axial direction (in the distal direction and in the opposite (proximal) direction inside the body lumen) is realized by driving a motor for axial movement operation 404, by rotating a ball screw 401 and by operating a support portion 403, that supports the scanner unit 300, in the axial direction.

The pull-back unit 310 is provided with a moving amount detector 405 for detecting the operation of the motor for axial movement operation 404 and for calculating the moving amount of the scanner unit 300 in the axial forward direction. In this embodiment representing one example of the disclosed apparatus, a two-phase encoder is used as the moving amount detector 405.

The pull-back unit 310 is further provided with a switch sensor (detection unit) 402. The switch sensor 402 is arranged and configured to detect the support portion 403 when the abutment unit 213 reaches a position of distance L from the sheath proximal end 212. That is, the switch sensor 402 is a sensor for detecting that the scanner unit 300 moved forward up to the position spaced a distance L from the forward end.

The control signal output for controlling the operation of the motor for axial movement operation 404, and signal inputs from the moving amount detector 405 and the switch sensor 402, are all carried out by an axial movement operation control unit 410.

The axial movement operation control unit 410is provided with a motor driver 411 and outputs a motor driving electric-current based on an instruction from the speed adjuster circuit 412 to the motor for axial movement operation 404. Also, a pulse signal from the moving amount detector 405 is received therein and is transmitted to the speed adjuster circuit 412.

The speed adjuster circuit 412 compares a speed set value transmitted from a speed set unit 414 and the pulse signal transmitted through the motor driver 411, and carries out a speed control for the movement in the axial direction of the scanner unit by instructing increase and decrease of the motor rotation speed with respect to the motor driver 411.

In a sequence control unit 415, when the automatic forward-movement button 321 of the operation unit 320 is pressed, this is recognized and an automatic forward-movement control process is executed. Specifically, based on the output from the switch sensor 402, a speed set value is set for the speed set unit 414 and concurrently, the motor driver 411 is instructed to start the operation of the motor for axial movement operation 404. Also, in accordance with the judgment result from an electric current judgment circuit (recognition unit) 413 which monitors a motor driving electric-current outputted by the motor driver 411, the motor driver 411 is instructed to stop the operation of the motor for axial movement operation 404.

In the sequence control unit 415, it is understood that it is configured to also control the operation of the motor for axial movement operation 404 when a button other than the automatic forward button 321 is pressed. But the description which follows is based on the automatic forward-movement control process.

### Flow of Automatic Forward-Movement Control Process in Pull-back Unit

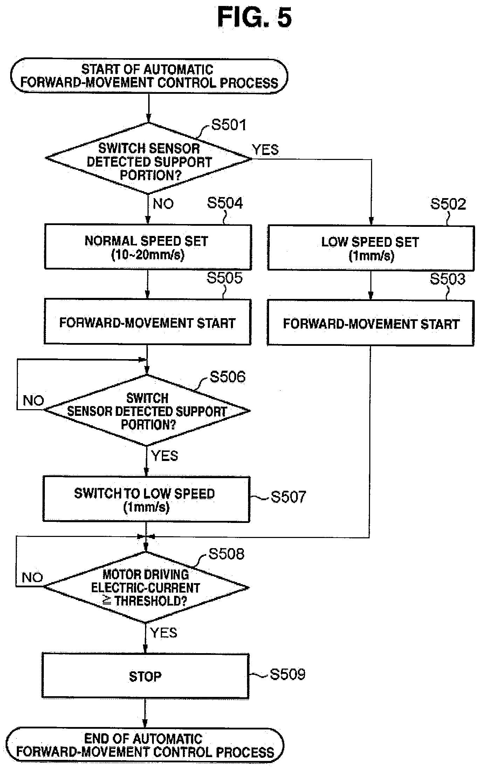

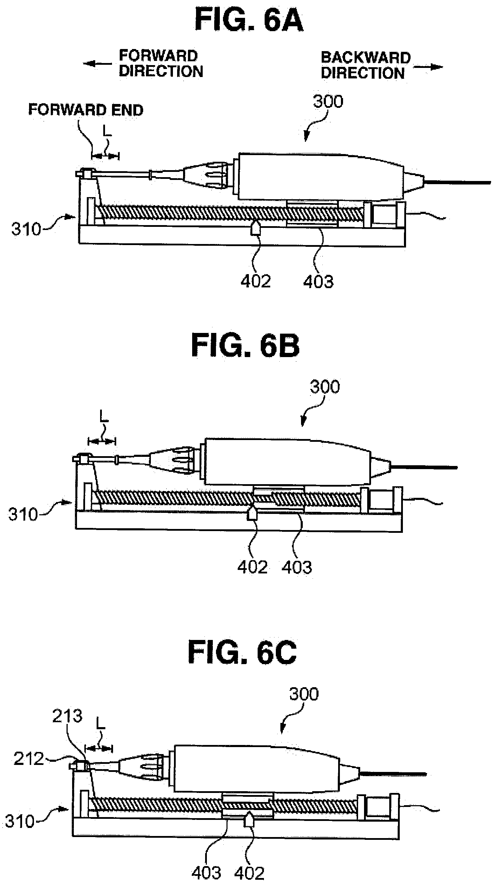

FIGS. 5 and 6A to 6C illustrate an example of the flow of the automatic forward-movement control process in the pull-back unit 310. FIG. 5 illustrates the automatic forward-movement control process in the pull-back unit 310 and FIGS. 6A to 6C illustrate an aspect of the axial movement operation of the scanner unit in the automatic forward-movement control process.

When the automatic forward button 321 is pressed, the execution of the automatic forward-movement control process shown in FIG. 5 is started. In step S501, it is judged whether or not the switch sensor 402 detects the support portion 403. At a point in time when the automatic forward button 321 is pressed and in a case in which the switch sensor 402 has detected the support portion 403, it is determined or judged that the scanner unit 300 lies in a position within the distance L (for example, 5mm) from the forward end, so that the process proceeds to step S502.

In step S502, the sequence control unit 415 sets a speed set value of a relatively lower speed (for example, 1mm/sec) with respect to the speed set unit 414. Further, in step S503, the motor driver 411 is instructed to operate axially in the forward direction according to the set speed value. Thus, the scanner unit 300 starts an axial movement operation at a lower speed or relatively low speed in the forward direction.

On the other hand, in step S501, in a case in which it is judged that the switch sensor 402 does not detect the support portion 403, it is determined that the scanner unit 300 lies in a position away from or spaced from the forward end by an amount of more than the distance L (for example, 5mm), so that the process proceeds to step S504 (see FIG. 6A).

In step S504, the sequence control unit 415 sets a speed set value of a normal speed set (for example, 10mm/sec to 20mm/sec) with respect to the speed set unit 414. Further, in step S505, the motor driver 411 is instructed to operate or move axially in the forward direction by that speed set value. Thus, the scanner unit 300 starts the axial movement operation at a normal speed in the forward direction.

In step S506, once again, it is judged whether or not the switch sensor 402 has detected the support portion 403. In step S506, in a case in which it is judged that the switch sensor 402 has not detected the support portion 403, the process stands by until the judgment that it has detected the support portion 403. In other words, as long as the determination at step S506 is NO, the axial movement operation toward the forward direction by a normal speed set continues.

On the other hand, in a case in which it is judged in step S506 that the switch sensor 402 has detected the support portion 403, it is determined that the scanner unit 300, which was in a position away from or spaced from the forward end by more than the distance L (for example, 5mm), has reached the position spaced the distance L, so that the process proceeds to step S507 (see FIG. 6B).

In step S507, the sequence control unit 415 switches the speed set value of a normal speed (for example, 10mm/sec to 20mm/sec), which has been set with respect to the speed set unit 414, to a speed set value of a lower speed or relatively low speed (for example, 1mm/sec).

Further, in step S508, the motor driving electric-current is monitored to determine whether the motor driving electric-current has reached a predetermined threshold or more. In a case in which it is judged in step S508 that the electric-current has not reached or exceeded the threshold, is judged that the scanner unit 300 has not reached the forward end and the process stands by. In other words, axial movement operation toward the forward direction at the lower speed set continues.

On the other hand, when it is judged in step S508 that the electric-current has reached or exceeded the threshold, it is judged that the axial movement operation toward the forward direction of the scanner unit 300 is suppressed (stopped) by a mechanism in which the abutment unit 213 of the scanner unit 300 abuts the sheath proximal end 212.

In the speed adjuster circuit 412, there is an instruction so as to increase the motor rotation speed in a case in which the speed of the axial movement operation toward the forward direction of the scanner unit 300, which is calculated based on the pulse signal outputted from the moving amount detector 405 with respect to the speed set value transmitted from the speed set unit 414, is relatively low. In this case, the motor driver increases the driving electric-current. Thus, in a case in which the axial movement operation toward the forward direction of the scanner unit 300 is suppressed, a situation arises in which the motor driving electric-current is going to increase.

Therefore, in a case in which the motor driving electric-current has reached or exceeded a predetermined threshold, it is possible to judge that it is in a state in which the axial movement operation toward the forward direction is suppressed, in other words, a state in which the scanner unit 300 reached the forward end.

In step S509, the sequence control unit 415 generates an instruction with respect to the motor driver 411 to stop the axial movement operation in the forward direction, and the automatic forward-movement control process ends.

As clear from the explanation above, in the scanner & pull-back unit 102 relating to this embodiment disclosed as an example, a construction is employed in which the switch sensor is arranged to switch the speed set value at a predetermined distance on the near side of the forward end at the time of the forward-movement operation of the scanner unit. Also, there was employed a construction in which the axial movement operation toward the forward direction is carried out at a normal speed until the scanner unit reaches a predetermined distance on the near side of the forward end (i.e., a predetermined distance from the forward-most position of the scanner unit) and after reaching the predetermined distance, the axial movement operation at a lower speed or relatively low speed is carried out.

Thus, it became possible to stop the scanner unit at the forward end without significantly increasing the moving period of time up to the forward end and at the same time, without damaging the optical probe unit.

The above-described first embodiment disclosed by way of example employs a construction which judges that the scanner unit 300 has reached the forward end (forward-most end) by monitoring the motor driving electric-current. This is due to the use of a DC motor as the motor for axial movement operation 404 in which the torque of the motor is in proportion to the motor driving electric-current and is due to the fact that in case of carrying out the speed control which is operated by a speed set value, there is utilized a characteristic that the motor driving electric-current is controlled in response to the variation of the load torque. However, the present invention is not limited in this regard and it is possible to employ a construction in which there is separately provided a sensor (for example, load-cell or the like) for detecting the pressure and in which that the scanner unit 300 has reached the forward end is judged based on the output of the sensor.

The above-described embodiments cite optical imaging apparatuses for diagnosis as examples, but the present invention is not to be limited by these as the disclosure here is also applicable to an ultrasound imaging apparatus for diagnosis, for example, to an intravascular ultrasound (IVUS) diagnostic apparatus.

The detailed description above describes features and aspects of embodiments of a motor drive apparatus and imaging apparatus for diagnosis disclosed by way of example. The invention is not limited, however, to the precise embodiments and variations described. Various changes, modifications and equivalents could be effected by one skilled in the art without departing from the scope of the invention as defined in the appended claims. It is expressly intended that all such changes, modifications and equivalents which fall within the scope of the claims are embraced by the claims.

## Claims

1. A motor drive apparatus (102) mounted with a probe unit (101) that includes a transmitting and receiving unit (221) which is configured to carry out signal transmission and reception continuously, the motor drive apparatus (102) comprising:
a scanner unit (300) connected to the transmitting and receiving unit (221), and configured to rotate the transmitting and receiving unit (221);
a scanner moving unit connected to the scanner unit (300) and configured to axially move the scanner unit (300) between a forward-most end position and a backward-most end position to move the transmitting and receiving unit (221) in an axial forward direction of the probe unit (101);
a detection unit (402) configured to detect that the scanner unit (300) has reached a position spaced from the forward-most end position of the scanner unit (300) by a predetermined distance when the scanner unit (300) is being operated in the axial forward direction; and
the scanner moving unit (310) comprising a control unit (111) which is configured to carry out speed control of the scanner unit (300) according to a first speed set value for movement of the scanner unit (300) in the axial forward direction towards the forward-most end position before the scanner unit (300) reaches the position spaced from the forward-most end position of the scanner unit (300) by the predetermined distance, and which is configured to carry out the speed control according to a second speed set value, less than the first speed set value, for movement of the scanner unit (300) toward the forward-most end position after the detection unit (402) has detected that the scanner unit (300) has reached the position spaced from the forward-most end position of the scanner unit (300) by the predetermined distance.

2. The motor drive apparatus (102) according to Claim 1, further comprising a recognition unit (413) for recognizing that the scanner unit (300) has reached the forward-most end position, and the control unit being configured to stop the movement of the scanner unit (300) in the axial forward direction, when the scanner unit (300) has reached the forward-most end position.

3. The motor drive apparatus (102) according to Claim 1 or 2, wherein the control unit (111) is configured to monitor a motor driving electric-current outputted with respect to a motor which axially moves the scanner unit (300) and to recognize that the scanner unit (300) has reached the forward-most end position when the motor driving electric-current reaches or exceeds a predetermined threshold.

4. The motor drive apparatus (102) according to Claim 1 or 3, further comprising an instruction unit (321) configured to permit input of an instruction for operating the scanner unit (300) in the axial forward direction, and when an instruction is inputted in the instruction unit (321), the control unit (111) controls the scanner unit (300) to move in the axial forward direction.

5. An imaging apparatus for diagnosis comprising the motor drive apparatus (102) according to Claim 1, configured to generate a plurality of cross-sectional images in an axial direction inside a body lumen based on line data generated from signals transmitted inside said body lumen and received as reflected signals through the motor drive apparatus (102).

## Patentansprüche

1. Motorantriebsvorrichtung (102), an der eine Sondeneinheit (101) angebracht ist, die eine Sende-und Empfangseinheit (221) beinhaltet, welche konfiguriert ist, um Signalübertragung und Signalempfang kontinuierlich durchzuführen, wobei die Motorantriebsvorrichtung (102) umfasst:
eine Scannereinheit (300), die mit der Sende- und Empfangseinheit (221) verbunden und konfiguriert ist, um die Sende- und Empfangseinheit (221) zu drehen;
eine Scanner-Bewegungseinheit, die mit der Scannereinheit (300) verbunden und konfiguriert ist, um die Scannereinheit (300) axial zwischen einer vordersten Endposition und einer hintersten Endposition zu bewegen, um die Sende- und Empfangseinheit (221) in einer axialen Vorwärtsrichtung der Sondeneinheit (101) zu bewegen;
eine Erfassungseinheit (402), die konfiguriert ist, um zu erfassen, dass die Scannereinheit (300) eine Position erreicht hat, die von der vordersten Endposition der Scannereinheit (300) um einen vorbestimmten Abstand beabstandet ist, wenn die Scannereinheit (300) in der axialen Vorwärtsrichtung betrieben wird; und
die Scanner-Bewegungseinheit (310) eine Steuereinheit (111) umfasst, die konfiguriert ist, um eine Geschwindigkeitssteuerung der Scannereinheit (300) gemäß einem ersten Geschwindigkeitssollwert für die Bewegung der Scannereinheit (300) in der axialen Vorwärtsrichtung in Richtung der vordersten Endposition durchzuführen, bevor die Scannereinheit (300) die Position erreicht, die von der vordersten Endposition der Scannereinheit (300) durch den vorbestimmten Abstand beabstandet ist, und die konfiguriert ist, um die Geschwindigkeitssteuerung gemäß einem zweiten Geschwindigkeitssollwert, der kleiner als der erste Geschwindigkeitssollwert ist, zum Bewegen der Scannereinheit (300) in Richtung der vordersten Endposition auszuführen, nachdem die Erfassungseinheit (402) erfasst hat, dass die Scannereinheit (300) die Position erreicht hat, die durch den vorbestimmten Abstand von der vordersten Endposition der Scannereinheit (300) beabstandet ist.

2. Motorantriebsvorrichtung (102) nach Anspruch 1, ferner umfassend eine Erkennungseinheit (413) zum Erkennen, dass die Scannereinheit (300) die vorderste Endposition erreicht hat, und die Steuereinheit konfiguriert ist, um die Bewegung der Scannereinheit (300) in der axialen Vorwärtsrichtung zu stoppen, wenn die Scannereinheit (300) die vorderste Endposition erreicht hat.

3. Motorantriebsvorrichtung (102) nach Anspruch 1 oder 2, wobei die Steuereinheit (111) konfiguriert ist, um einen elektrischen Motorantriebsstrom zu überwachen, der in Bezug auf einen Motor abgegeben wird, der die Scannereinheit (300) axial bewegt, und um zu erkennen, dass die Scannereinheit (300) die vordere Endposition erreicht hat, wenn der elektrische Motorantriebsstrom einen vorbestimmten Schwellenwert erreicht oder überschreitet.

4. Motorantriebsvorrichtung (102) nach Anspruch 1 oder 3, ferner umfassend eine Anweisungseinheit (321), die konfiguriert ist, um die Eingabe einer Anweisung zum Betreiben der axialen Vorwärtsrichtung der Scannereinheit (300) zu ermöglichen, und wenn eine Anweisung in die Anweisungseinheit (321) eingegeben wird, steuert die Steuereinheit (111) die Scannereinheit (300), um sich in der axialen Vorwärtsrichtung zu bewegen.

5. Abbildungsvorrichtung zur Diagnose, umfassend die Motorantriebsvorrichtung (102) nach Anspruch 1, die konfiguriert ist, um eine Vielzahl von Querschnittsbildern in einer axialen Richtung innerhalb eines Körperlumens zu erzeugen, basierend auf Zeilendaten, die aus Signalen erzeugt werden, die innerhalb des Körperlumens übertragen und als reflektierte Signale durch die Motorantriebsvorrichtung (102) empfangen werden.

## Revendications

1. Appareil d'entraînement à moteur (102) équipé d'une unité de sonde (101) qui comprend une unité d'émission et de réception (221) qui est configurée pour réaliser une émission et une réception de signaux de façon continue, l'appareil d'entraînement à moteur (102) comprenant :
une unité de balayage (300) reliée à l'unité d'émission et de réception (221), et configurée pour faire tourner l'unité d'émission et de réception (221) ;
une unité de déplacement d'unité de balayage reliée à l'unité de balayage (300) et configurée pour déplacer axialement l'unité de balayage (300) entre une position extrême la plus en avant et une position extrême la plus en arrière afin de déplacer l'unité d'émission et de réception (221) dans une direction avant axiale de l'unité de sonde (101) ;
une unité de détection (402) configurée pour détecter que l'unité de balayage (300) a atteint une position espacée d'une distance prédéterminée de la position extrême la plus en avant de l'unité de balayage (300) lorsque l'unité de balayage (300) est actionnée dans la direction avant axiale ; et
l'unité de déplacement d'unité de balayage (310) comprenant une unité de commande (111) qui est configurée pour réaliser une régulation de vitesse de l'unité de balayage (300) conformément à une première valeur de consigne de vitesse pour le mouvement de l'unité de balayage (300) dans la direction avant axiale vers la position extrême la plus en avant avant que l'unité de balayage (300) n'atteigne la position espacée de la distance prédéterminée de la position extrême la plus en avant de l'unité de balayage (300), et qui est configurée pour réaliser la régulation de vitesse conformément à une deuxième valeur de consigne de vitesse, inférieure à la première valeur de consigne de vitesse, pour le mouvement de l'unité de balayage (300) vers la position extrême la plus en avant après que l'unité de détection (402) a détecté que l'unité de balayage (300) a atteint la position espacée de la distance prédéterminée de la position extrême la plus en avant de l'unité de balayage (300).

2. Appareil d'entraînement à moteur (102) selon la revendication 1, comprenant en outre une unité de reconnaissance (413) pour reconnaître que l'unité de balayage (300) a atteint la position extrême la plus en avant, l'unité de commande étant configurée pour arrêter le mouvement de l'unité de balayage (300) dans la direction avant axiale lorsque l'unité de balayage (300) a atteint la position extrême la plus en avant.

3. Appareil d'entraînement à moteur (102) selon la revendication 1 ou 2, dans lequel l'unité de commande (111) est configurée pour surveiller un courant électrique d'attaque de moteur délivré relativement à un moteur qui déplace axialement l'unité de balayage (300) et pour reconnaître que l'unité de balayage (300) a atteint la position extrême la plus en avant lorsque le courant électrique d'attaque de moteur atteint ou dépasse un seuil prédéterminé.

4. Appareil d'entraînement à moteur (102) selon la revendication 1 ou 3, comprenant en outre une unité d'instruction (321) configurée pour permettre l'introduction d'une instruction d'actionnement de l'unité de balayage (300) dans la direction avant axiale, et lorsqu'une instruction est introduite dans l'unité d'instruction (321), l'unité de commande (111) commande le déplacement de l'unité de balayage (300) dans la direction avant axiale.

5. Appareil d'imagerie diagnostique comprenant l'appareil d'entraînement à moteur (102) selon la revendication 1, configuré pour générer une pluralité d'images de coupe dans une direction axiale à l'intérieur d'une lumière corporelle sur la base de données de ligne générées à partir de signaux émis à l'intérieur de ladite lumière corporelle et reçus sous forme de signaux réfléchis au moyen de l'appareil d'entraînement à moteur (102).
